(19) Europäisches Patentamt
European Patent Office
Office européen des brevets

(11) Numéro de publication : **0 032 275**
**B1**

# (12) FASCICULE DE BREVET EUROPÉEN

(45) Date de publication du fascicule du brevet : **25.07.84**

(21) Numéro de dépôt : **80201248.4**

(22) Date de dépôt : **31.12.80**

(51) Int. Cl.³ : **C 07 C 49/84**, C 07 C 49/83, C 07 C 45/71, C 07 C 45/43, C 07 C 45/00

## (54) Procédé pour la fabrication de 2-hydroxybenzophénones substituées.

(30) Priorité : **11.01.80 FR 8000668**

(43) Date de publication de la demande : **22.07.81 Bulletin 81/29**

(45) Mention de la délivrance du brevet : **25.07.84 Bulletin 84/30**

(84) Etats contractants désignés : **AT BE CH DE FR GB IT LI NL SE**

(56) Documents cités :
**DE-A- 2 451 037**
**GB-A- 1 246 958**
**US-A- 3 769 349**

(73) Titulaire : **AMERICAN CYANAMID COMPANY**
**1937 West Main Street P.O. Box 60**
**Stamford Connecticut 06904 (US)**

(72) Inventeur : **Paucot, André**
**Avenue Général Eisenhower, 63**
**B-1030 Bruxelles (BE)**
Inventeur : **Malfroid, Pierre**
**Rue de la Taille, 19**
**B-5790 Jemeppe-sur-Sambre (BE)**
Inventeur : **Mulders, Julien**
**Beukenlaan, 21**
**B-1512 Dworp (BE)**

(74) Mandataire : **Wächtershäuser, Günter, Dr.**
**Tal 29**
**D-8000 München 2 (DE)**

**EP 0 032 275 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

L'invention concerne un procédé pour la fabrication de 2-hydroxybenzophénones substituées de formule

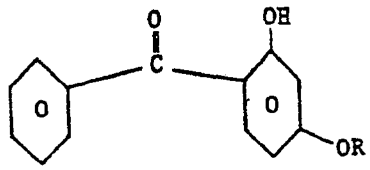

où R est un radical aliphatique non substitué et saturé, en faisant réagir en une première étape du trichlorométhylbenzène avec le m-dihydroxybenzène en présence d'un solvant et en alcoylant en une deuxième étape le 2,4-dihydroxybenzophénone obtenu.

Il est connu, par le brevet Etats-Unis 3 639 483 déposé le 8 juillet 1968 et cédé à FURUKAWA ELECTRIC Co Ltd, de fabriquer des 2-hydroxy-4-alkoxybenzophénones par deux réactions successives faisant intervenir du trichlorométhylbenzène, de m-dihydroxybenzène, de l'eau, des alcools et des halogénures d'alkyle. La première réaction vise à obtenir la 2,4-dihydroxybenzophénone en faisant réagir du trichlorométhylbenzène avec le m-dihydroxybenzène dans un milieu solvant constitué d'eau et d'un alcool contenant moins de quatre atomes de carbone, et de préférence du méthanol. La 2,4-dihydroxybenzophénone est ensuite mise à réagir dans un autre milieu solvant, constitué d'une cétone aliphatique cyclique, avec un halogénure d'alkyle en présence d'un carbonate alcalin de façon à former la 2-hydroxy-4-alkoxybenzophénone souhaitée.

Dans le brevet belge 795 782 déposé le 22 février 1973 au nom de BAYER AG, on décrit un procédé analogue dans lequel on opère également en deux étapes avec des solvants différents. A la première étape, le solvant est constitué d'un composé organique non miscible à l'eau et d'un alcool aliphatique inférieur contenant moins de six atomes de carbone dans sa molécule qui est de préférence l'éthanol ou le méthanol. L'emploi de ce solvant permettrait notamment d'éviter les inconvénients provenant de la formation d'acide benzoïque résultant de la réaction entre le trichlorométhylbenzène et l'eau. A la deuxième étape, on met en œuvre des solvants, tels que les cétones, différents de ceux utilisés à la première étape.

Ces procédés connus de fabrication des 2-hydroxy-4-alkoxybenzophénones sont difficiles à mettre en œuvre. En effet, l'emploi de solvants différents à la première et à la deuxième étape, implique une série d'opérations de séparation des produits, de séchage et de distillation entre les deux étapes qui entraînent une perte en rendement et en énergie.

La présente invention vise à éviter ces inconvénients des procédés connus et à fournir un procédé qui permette la fabrication de 2-hydroxy-4-alkoxybenzophénones, à partir de trichlorométhylbenzène et de m-dihydroxybenzène, dans un milieu réactionnel unique.

L'invention concerne à cet effet un procédé pour la fabrication de 2-hydroxybenzophénones substituées de formule

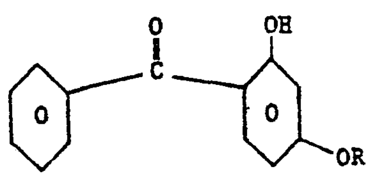

où R est un radical aliphatique non substitué et saturé, en faisant réagir en une première étape du trichlorométhylbenzène avec le m-dihydroxybenzène en présence d'un solvant et en alcoylant en une deuxième étape le 2,4-dihydroxybenzophénone obtenu, caractérisé en ce que le solvant contient un composé de formule ROH où R a la signification ci-dessus et que la deuxième étape est exécutée par l'addition d'un agent basique sans séparation du produit de la première étape.

Le solvant mis en œuvre dans le procédé selon l'invention comprend un ou plusieurs alcools de formule ROH où R a une signification qui sera donnée ci-après.

Outre les alcools précités, le solvant peut éventuellement contenir d'autres composés tels que de l'eau ou des composés organiques aliphatiques ou aromatiques tels que les hydrocarbures cycliques ou acycliques contenant de 5 à 12 atomes de carbone, comme le décane ou le cyclohexane, les hydrocarbures halogénés contenant de 1 à 10 atomes de carbone comme le 1,2-dichloroéthane et les hydrocarbures aromatiques, substitués ou non tels que le toluène ou l'isopropylbenzène.

Habituellement la composition du solvant est choisie de façon à permettre la solubilisation de tous les réactifs mis en œuvre. On préfère travailler avec un solvant qui est constitué à raison d'au moins 10 %

en poids d'un composé ROH tel que défini ci-avant. Les meilleurs résultats ont été obtenus avec des solvants constitués d'au moins 25 % en poids de tels composés ROH et de 0 à 75 % au plus en poids d'eau.

La quantité de solvant mise en œuvre pour réaliser le procédé selon l'invention, peut varier dans de larges limites, qui sont les mêmes que celles préconisées dans les procédés connus et qui se situent généralement entre 1 à 10 parties en poids par rapport au trichlorométhylbenzène mis en œuvre.

Le procédé selon l'invention peut avantageusement être réalisé en deux stades : dans un premier stade, tout le trichlorométhylbenzène et le m-dihydroxybenzène sont mis à réagir dans le milieu réactionnel sans addition de catalyseur et selon un schéma réactionnel connu en soi, par les publications antérieures, de façon à former le 2,4-dihydroxybenzophénone. A ce stade, tout le solvant peut se trouver dans le milieu réactionnel dès le début de la réaction ou peut y être introduit au fur et à mesure de l'avancement de la réaction.

Les quantités des réactifs mises en œuvre ne sont pas critiques en elles-mêmes et leur rapport molaire varie en général dans l'intervalle de 1 : 9 et 9 : 1. Habituellement on travaille toutefois avec un léger excès de trichlorométhylbenzène de sorte que le mélange réactionnel contienne de 1 à 1,5 mole du trichlorométhylbenzène par mole de m-dihydroxybenzène.

Les autres paramètres tels que l'ordre d'introduction des réactifs et la température de réaction ne sont pas critiques en eux-mêmes non plus. La température de réaction est choisie en général entre 50 et 180 °C. Habituellement, on préfère travailler à une température située aux environs du point d'ébullition du mélange réactionnel ou légèrement en dessous de celui-ci.

Pour déclencher le deuxième stade de la réaction, dans lequel on réalise l'alcoylation de la 2,4-dihydroxybenzophénone, il suffit d'ajouter au milieu réactionnel un agent basique sans qu'il soit nécessaire de séparer les réactifs du solvant utilisé au premier stade et sans qu'il soit nécessaire d'ajouter un quelconque autre réactif supplémentaire tel qu'un halogénure d'alkyle lorsque le composé ROH a été mis en œuvre en quantité relativement importante au premier stade de la réaction. On préfère cependant ajouter un halogénure aliphatique au second stade. Celui-ci répond à la formule RX où X représente le brome ou, de préférence, le chlore et R est le radical présent dans la 2-hydroxybenzophénone substituée que l'on fabrique et dans le composé de formule ROH.

Généralement, le radical R est un radical aliphatique non substitué et saturé contenant de 3 à 15 atomes de carbone. De préférence, le radical R contient de 5 à 12 atomes de carbone. Les meilleurs résultats ont été obtenus avec les radicaux aliphatiques linéaires. Tout particulièrement préférés sont les radicaux aliphatiques linéaires contenant de 7 à 10 atomes de carbone et plus particulièrement le radical n-octyle.

La quantité de composé RX à mettre en œuvre n'est pas critique en elle-même. Elle dépend de la nature et de la quantité de composé ROH présent dans le solvant et de la quantité de 2,4-dihydroxybenzophénone formée après la première étape. Dans le cas où un solvant contenant uniquement un composé ROH est utilisé, l'addition d'halogénure d'alkyle à la deuxième étape est relativement faible.

La nature de l'agent basique n'est pas critique en elle-même. Habituellement, on utilise une base alcaline telle que l'hydroxyde de sodium ou un sel alcalin basique tel que le carbonate ou le bicarbonate de sodium ou de potassium. L'agent basique préféré est le bicarbonate de sodium. L'adjonction de l'agent basique est en général accompagnée de l'addition de quantités catalytiques de sels alcalins halogénés (iodure ou bromure de sodium ou de potassium par exemple).

La quantité d'agent basique ajoutée à ce stade, est la même que celles préconisées dans les procédés connus. Habituellement, on ajoute 1,0 à 1,5 mole par mole de 2,4-dihydroxybenzophénone que contient théoriquement le milieu après le premier stade de la réaction.

L'agent basique et les quantités catalytiques de sels alcalins halogénés peuvent être ajoutés en une fois, en plusieurs fois, ou encore en continu, suivant l'allure que l'on désire imposer au deuxième stade de la réaction.

L'agent basique et, le cas échéant, le composé RX peuvent être ajoutés au mélange réactionnel à tout moment après que la 2,4-dihydroxybenzophénone a commencé à se former. On préfère toutefois pour des raisons de cinétique, de technologie et de rendement n'ajouter ces composés qu'après que la réaction de formation de la 2,4-dihydroxybenzophénone soit terminée. Par ailleurs, ces composés peuvent être ajoutés en une seule fois ou par portions.

La température à laquelle on réalise le deuxième stade de la réaction est généralement située entre les mêmes limites que celles du premier stade. Avantageusement, ce deuxième stade se fait également à des températures situées aux environs du point d'ébullition.

Après que la réaction est terminée, la 2-hydroxybenzophénone substituée peut être séparée du mélange réactionnel par tout moyen utilisé habituellement à cet effet. Une façon avantageuse de procéder, consiste à traiter d'abord le mélange réactionnel avec une solution aqueuse chaude de façon à extraire les sels présents qui sont solubles dans l'eau. La phase organique résiduaire peut ensuite être soumise à des opérations de distillation ou de cristallisation, ce qui permet de récupérer les différents composants du solvant, ainsi que la 2-hydroxybenzophénone substituée.

Les 2-hydroxybenzophénones substituées obtenues selon l'invention peuvent être utilisées dans les différents domaines d'application qui leur sont propres sans autre opération d'épuration.

Une utilisation particulièrement intéressante de ces produits réside dans l'exploitation de leurs propriétés d'absorption de la lumière ultraviolette pour stabiliser des matières plastiques diverses

obtenues par polycondensation ou par polymérisation radicalaire en chaîne telles que le chlorure de polyvinyle, le polyéthylène et le polypropylène.

Les exemples ci-après sont donnés pour illustrer l'invention.

Exemple 1

Cet exemple est donné à titre de comparaison.

Etape 1 — Fabrication de la 2,4-dihydroxybenzophénone

Dans un ballon de 500 $cm^3$ à trois tubulures, muni d'un agitateur, d'un thermomètre et d'un réfrigérant, on dissout 50,87 g de trichlorométhylbenzène, pur à 98 %, dans 180 $cm^3$ de toluène pur pour analyses. Le tout est chauffé à 60 °C et on ajoute goutte à goutte et en 1 heure, une solution de 22,07 g de m-dihydroxybenzène, pur à 98 %, dissous dans un mélange constitué de 14 g de méthanol et de 20 $cm^3$ de toluène, tout en maintenant la température à 60 °C. Le mélange réactionnel est ensuite agité pendant 6 heures à cette même température. On laisse ensuite refroidir le mélange à température ambiante et reposer pendant une nuit.

Après filtration sur filtre G-4 et séchage sous vide on récupère ensuite 32,6 g de 2,4-dihydroxybenzophénone, ce qui constitue un rendement de 87 % par rapport à la valeur théorique de la quantité de 2,4-dihydroxybenzène mise en œuvre.

Etape 2 — Fabrication de la 2-hydroxy-4-octoxybenzophénone

Dans un ballon de 250 $cm^3$ à trois tubulures muni d'un agitateur, d'un thermomètre et d'un réfrigérant, on introduit 100 $cm^3$ de cyclohexanone p.p.a., 11,87 g de la 2,4-dihydroxybenzophénone synthétisé à l'étape 1 et 9,11 g de chlorure d'octyle pur à 99 %, 7,2 g de bicarbonate de soude et 0,83 g de KI. Après 5 heures de réaction dans des conditions de reflux et pendant lesquelles la température varie entre 142 °C et 135 °C, on sépare l'insoluble par filtration avec succion sur filtre G-4. Le filtrat est ensuite évaporé et le résidu est recristallisé dans 40 $cm^3$ de méthanol sec et l'on obtient finalement 14,85 g de 2-hydroxy-4-octyloxybenzophénone pur ce qui représente un rendement de 82 % par rapport à la valeur théorique prévue.

Le rendement total par rapport à la quantité de 2,4-dihydroxybenzène mise en œuvre à l'étape 1 est donc d'environ 70 %.

Exemple 2

Dans un autoclave de 2 $m^3$, on introduit 600 kg de n-octanol dans lesquels on dissout 250 kg (2,27 kmol) de m-dihydroxybenzène. La température est portée à 120 °C et on ajoute en 4 heures et sous agitation tout en maintenant la température initiale, 555 kg (2,84 kmol) de trichlorométhylbenzène.

Au mélange réactionnel ainsi obtenu, on ajoute ensuite 348 kg de n-octanol, 17 kg d'iodure de potassium et 100 kg de bicarbonate de sodium. La température est ensuite portée en 5 heures, pendant lesquelles on rajoute encore 150 kg de bicarbonate de sodium, à 160 °C.

Après lavage à l'eau et évaporation de la phase organique liquide on obtient finalement un produit solide contenant 530 kg de 2-hydroxy-4-octyloxybenzophénone ce qui correspond à un rendement molaire par rapport au 2,4-dihydroxybenzène mis en œuvre qui est également d'environ 70 %.

Exemple 3

Dans un autoclave de 3 $m^3$ contenant une solution homogène, agitée, constituée de 400 kg (3,63 kmol) de m-dihydroxybenzène, de 281 kg (2,18 kmol) de n-octanol et de 262 kg (14,53 kmol) d'eau, chauffée à 90 °C, on introduit progressivement et pendant 2 heures 746 kg (3,81 kmol) de trichlorométhylbenzène.

Au mélange réactionnel ainsi obtenu, on ajoute ensuite 662 kg (5,09 kmol) de n-octanol, 850 kg (5,72 kmol) de n-chlorooctane, 28 kg d'iodure de potassium et 400 kg de bicarbonate de sodium. La température est portée en 2 heures à 160 °C et maintenue pendant 4 heures à cette valeur.

Après lavage à l'eau et évaporation de la phase organique liquide on obtient finalement 883 kg de 2-hydroxy-4-octyloxybenzophénone solide ce qui correspond à un rendement molaire par rapport au 2,4-dihydroxybenzophénone mis en œuvre d'environ 75 %.

Il ressort de la comparaison des exemples 1, 2 et 3 que le procédé selon l'invention permet d'atteindre des performances égales à celles des procédés connus sans qu'il soit nécessaire d'isoler un produit intermédiaire.

**Revendications**

1. Procédé pour la fabrication de 2-hydroxybenzophénones substituées de formule

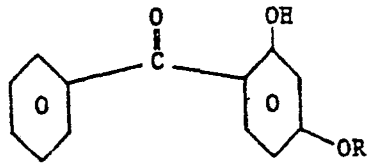

où R est un radical aliphatique non substitué et saturé, en faisant réagir en une première étape du trichlorométhylbenzène avec le m-dihydroxybenzène en présence d'un solvant et en alcoylant en une deuxième étape le 2,4-dihydroxybenzophénone obtenu, caractérisé en ce que le solvant contient un composé de formule ROH où R a la signification ci-dessus et que la deuxième étape est exécutée par l'addition d'un agent basique sans séparation du produit de la première étape.

2. Procédé selon la revendication 1, caractérisé en ce que le radical R contient de 3 à 15 atomes de carbone.

3. Procédé selon la revendication 2, caractérisé en ce que le radical R contient de 5 à 12 atomes de carbone.

4. Procédé selon la revendication 3, caractérisé en ce que le radical R est un radical aliphatique linéaire, contenant de 7 à 10 atomes de carbone.

5. Procédé selon la revendication 4, caractérisé en ce que le radical R est le n-octyle.

6. Procédé selon l'une quelconque des revendications 1 à 5, caractérisé en ce que le solvant est constitué d'au moins 25 % en poids de composé ROH et de 0 à 75 % au plus en poids d'eau.

7. Procédé selon l'une quelconque des revendications 1 à 6, caractérisé en ce que dans la seconde étape on ajoute en plus un halogénure d'alcoyle de formule RX où X est le brome ou le chlore.

8. Procédé selon la revendication 7, caractérisé en ce que X est le chlore.

**Claims**

1. Process for the preparation of substituted 2-hydroxybenzophenones of the formula

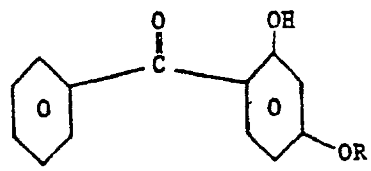

wherein R is a non-substituted saturated aliphatic group by reacting in a first stage trichloromethylbenzene with m-dihydroxybenzene in the presence of a solvent and alkylating in a second stage the obtained 2,4-dihydroxybenzophenone, characterized in that the solvent contains a compound of the formula ROH wherein R has the meaning given above and that the second stage is executed by the addition of a basic agent without separation of the product of the first stage.

2. A process according to Claim 1, characterized in that the group R contains from 3 to 15 carbon atoms.

3. A process according to Claim 2, characterized in that the group R contains from 5 to 12 carbon atoms.

4. A process according to Claim 3, characterized in that the group R is a linear aliphatic group containing from 7 to 10 carbon atoms.

5. A process according to Claim 4, characterized in that the group R is the n-octyl group.

6. A process according to one of Claims 1 to 5, characterized in that the solvent consists of at least 25 wt.-% ROH and 0 to 75 wt.-% water.

7. A process according to one of Claims 1 to 6, characterized in that in the second stage an alkyl halide of the formula RX is added, whereby X represents bromine or chlorine.

8. A process according to Claim 7, characterized in that X represents chlorine.

**Ansprüche**

1. Verfahren zur Herstellung von substituierten 2-Hydroxybenzophenonen der Formel

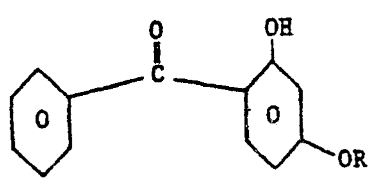

wobei R für eine nicht-substituierte gesättigte aliphatische Gruppe steht, wobei in einer ersten Stufe Trichlormethylbenzol mit m-Dihydroxybenzol in Gegenwart eines Lösungsmittels umgesetzt wird und in einer zweiten Stufe das erhaltene 2,4-Dihydroxybenzophenon alkyliert wird, dadurch gekennzeichnet, daß das Lösungsmittel eine Verbindung der Formel ROH enthält, wobei R die oben angegebene Bedeutung hat und daß man die zweite Stufe durchführt durch Zugabe eines basischen Mittels, ohne das Produkt der ersten Stufe abzutrennen.

2. Verfahren nach Anspruch 1, dadurch gekennzeichnet, daß die Gruppe R von 3 bis 15 Kohlenstoffatome enthält.

3. Verfahren nach Anspruch 2, dadurch gekennzeichnet, daß die Gruppe R von 5 bis 12 Kohlenstoffatome enthält.

4. Verfahren nach Anspruch 3, dadurch gekennzeichnet, daß die Gruppe R eine lineare aliphatische Gruppe ist, welche von 7 bis 10 Kohlenstoffatome enthält.

5. Verfahren nach Anspruch 4, dadurch gekennzeichnet, daß die Gruppe R die n-Octylgruppe ist.

6. Verfahren nach einem der Ansprüche 1 bis 5, dadurch gekennzeichnet, daß das Lösungsmittel aus mindestens 25 Gew.-% ROH und 0 bis 75 Gew.-% Wasser besteht.

7. Verfahren nach einem der Ansprüche 1 bis 6, dadurch gekennzeichnet, daß in der zweiten Stufe ein Alkylhalogenid der Formel RX zugegeben wird, wobei X für Brom oder Chlor steht.

8. Verfahren nach Anspruch 7, dadurch gekennzeichnet, daß X für Chlor steht.